# EUROPÄISCHE PATENTANMELDUNG

(11) **EP 4 202 098 A1**
(43) Veröffentlichungstag der Anmeldung: **28.06.2023**
(21) Anmeldenummer: 22210661.9
(22) Anmeldetag: 30.11.2022
(51) Int. Cl.: D04H 1/4266, A47L 13/16, B32B 5/26, D04H 1/4282, D04H 1/4291, D04H 1/4382, D04H 1/485, D04H 1/541

(54) **VERFAHREN ZUR HERSTELLUNG EINES NONWOVENELEMENTS FÜR HYGIENEARTIKEL**

(30) Priorität: 27.12.2021 DE 102021006353
(71) Anmelder: Nitto Advanced Nonwoven Ascania GmbH, 06449 Aschersleben (DE)
(72) Erfinder: TRINKAUS, Jan Michael, 53881 Euskirchen (DE); HUEPFEL, Raik, 06456 Arnstein (DE)
(74) Vertreter: Andrejewski - Honke Patent- und Rechtsanwälte Partnerschaft mbB

(57) **Zusammenfassung**

Verfahren zur Herstellung eines Nonwovenelements (13) für Hygieneartikel, umfassend zumindest die Schritte: Bildung einer Faserflorbahn (9) aus einem mehrschichtigen Nonwovenmaterial mit zumindest einer kardierten Stapelfaserlage und einer auf der Stapelfaserlage angeordneten Speicherlage (7), welche Zellstofffasern (5) aufweist, wobei zumindest ein Teil der Stapelfasern (4) der Stapelfaserlage thermoplastisch ausgebildet sind, Beaufschlagung der Faserflorbahn (9) mit Flüssigkeitsstrahlen, wodurch die Fasern des mehrschichtigen Nonwovenmaterials miteinander vermischt und verschlungen werden und der Faserflorbahn (9) eine Oberflächenstruktur eingeprägt wird, Beaufschlagung der Faserflorbahn (9) mit Hitze, wodurch die thermoplastischen Stapelfasern (4) zumindest teilweise aufschmelzen und die Faserflorbahn (9) zu einer Nonwovenbahn verfestigen, Abtrennen einzelner Nonwovenelemente (13) aus der Nonwovenbahn.

## Beschreibung

Die Erfindung betrifft ein Verfahren zur Herstellung eines Nonwovenelements für Hygieneartikel, insbesondere für Hygienetücher.

Hygienetücher dienen im Alltag der Reinigung von Oberflächen, wobei es sich hierbei sowohl um die Oberfläche von Gegenständen als auch um Körperoberflächen handeln kann. Ein typischer Anwendungsfall ist beispielsweise die Reinigung und Desinfektion von Händen, wobei dann die Hygienetücher üblicherweise in einem feuchten Zustand verwendet werden, in dem das entsprechende Hygienetuch zuvor und insbesondere bereits im Zuge der Herstellung mit einem Reinigungs- und/oder Desinfektionsmittel getränkt wurde. Alternativ können Hygienetücher auch als klassische Reinigungstücher zur Aufnahme von Flüssigkeiten, Staub oder Schmutz dienen, wobei dann auch der Einsatz in einem trockenen Zustand möglich ist. Hierbei werden die Hygienetücher auch als Teil von Reinigungsvorrichtungen verwendet. Dies umfasst beispielsweise die Verwendung an einem Saugroboter oder aber auch an einfachen Handgriffen, an denen dann die entsprechenden Hygienetücher montiert werden.

Bei der Verwendung von feuchten Hygienetüchern steht insbesondere das Bedürfnis im Vordergrund, die Reinigungsflüssigkeit innerhalb des Hygienetuches zu halten und bei Bedarf bzw. bei Ausübung von Druck an die Oberfläche abzugeben. Zugleich muss das Hygienetuch so beschaffen sein, dass der mit der Reinigungsflüssigkeit entfernte Schmutz in geeigneter Weise von dem Hygienetuch aufgenommen wird. Hierzu bedarf es insbesondere einer verhältnismäßig großen spezifischen Oberfläche, die üblicherweise einerseits durch die verwendeten Materialien und andererseits durch eine eingeprägte Oberflächenstruktur erreicht wird.

Vor diesem Hintergrund ist üblicherweise eine Schicht aus Zellstoff vorgesehen, welche auch als Pulp-Schicht bezeichnet wird. Diese Zellstoffschicht ist geeignet, verhältnismäßig große Mengen an Flüssigkeit aufzunehmen. Hierbei kann es sich beispielsweise bei feuchten Hygienetüchern um die Reinigungsflüssigkeit handeln. Aber auch bei der Verwendung von trockenen Reinigungstüchern kann eine solche Schicht sinnvoll sein, um Feuchtigkeit in geeigneter Weise von Oberflächen zu entfernen.

Diese Zellstoffschicht wird an einer weiteren Schicht angeordnet und befestigt, wobei es sich üblicherweise um eine Schicht aus Nonwovenmaterial handelt, da dieses Material einerseits kostengünstig herstellbar ist und andererseits eine ausreichende Reinigungsfähigkeit und Stabilität aufweist. Nonwovenmaterialien werden im Allgemeinen auch als Vliesstoff bezeichnet und unterscheiden sich von gewirkten oder gewebten Materialien durch eine im Wesentlichen lose Aneinanderreihung einzelner Fasern. Diese Aneinanderreihung einzelner Fasern wird auch als Faserflor bezeichnet, wobei dieser Faserflor in einem anschließenden Prozessschritt verfestigt werden muss, um hierdurch eine ausreichende Festigkeit und Stabilität auszubilden. Diese Verfestigung kann beispielsweise in einer mechanischen, thermischen oder chemischen Art und Weise erfolgen, wobei hierdurch die Fasern miteinander verbunden oder verschlungen werden.

Darüber hinaus wird bei Nonwovenmaterialien auch zwischen der Art der verwendeten Fasern unterschieden. Eine Möglichkeit besteht beispielsweise darin, Endlosfilamente zu einem sogenannten Spinnvlies zusammenzufassen. Diese Endlosfilamente werden üblicherweise gemeinsam extrudiert, wodurch sich bereits in einem gewissen Maße eine Anordnung in Produktionsrichtung ergibt. Allerdings sind für eine solche Art der Nonwovenbildung ausschließlich thermoplastische Fasermaterialien einsetzbar.

Alternativ ist es auch möglich, einzelne, kurze Fasern - welche auch als Stapelfasern bezeichnet werden - zu einem Stapelvlies zusammenzulegen, wobei hierzu die einzelnen Stapelfasern üblicherweise in einem sogenannten Kardierverfahren in Produktionsrichtung ausgerichtet werden.

Insgesamt zeichnen sich Spinnvliese aufgrund der langen Faserlänge durch eine wesentlich höhere Steifigkeit und Festigkeit aus, während Stapelvliese aufgrund der kurzen Faserlänge eine weiche und bauschigere Oberfläche ausbilden. Darüber hinaus können Stapelvliese auch aus nicht-thermoplastischen und damit nicht extrudierbaren Materialien gebildet werden. Hierdurch sind Stapelvliese sehr viel flexibler einsetzbar.

Verfahren zur Herstellung von Nonwovenelementen für Hygienetücher sind grundsätzlich aus dem Stand der Technik bekannt. So beschreibt beispielsweise die EP 1 775 116 B1 ein mehrschichtiges Nonwovenelement mit zwei äußeren Faserlagen, welche sowohl in Form von kardierten Stapelfaserlagen oder aber auch als Spinnfaserlagen ausgebildet sein können und welche eine Schicht aus Zellstoff einschließen. Dieser mehrschichtige Aufbau wird sodann durch Beaufschlagung von Wasserstrahlen miteinander verfestigt, wodurch sich insbesondere die Fasern der äußeren Schicht miteinander verbinden und so für eine ausreichende Festigkeit sorgen.

Auch die EP 2 010 703 B1 offenbart einen mehrschichtigen Aufbau mit zwei Faserflorlagen aus einem Vliesstoff, welche eine Schicht aus Zellstoff einschließen. Dieser Aufbau wird sodann ebenfalls über Beaufschlagung von Wasserstrahlen sowie durch ein sogenanntes Air-Through-Bonding (ATB) verfestigt. Beim Air-Through-Bonding werden insbesondere in den äußeren Lagen thermoplastische Materialien verwendet, welche unter Einwirkung von Hitze aufschmelzen und sich hierdurch miteinander verbinden. Neben dem Verschlingen der einzelnen Fasern, welches durch die Beaufschlagung mit Wasserstrahlen erreicht wird, ergibt sich somit eine zusätzliche Verfestigung, bei der die einzelnen Fasern stoffschlüssig miteinander verbunden werden.

Die bislang verwendeten Verfahren haben sich grundsätzlich bewährt. Jedoch besteht zunehmend Bedarf an Hygieneartikeln, welche einerseits besonders flexibel für unterschiedliche Anwendungsgebiete einsetzbar sind und welche sich darüber hinaus durch eine hohe Reinigungswirkung sowie eine gute Stabilität auszeichnen.

Vor diesem Hintergrund lehrt die Erfindung ein Verfahren zur Herstellung eines Nonwovenelements für Hygieneartikel gemäß Anspruch 1, ein Nonwovenelement für Hygieneartikel gemäß Anspruch 15 sowie einen Hygieneartikel gemäß Anspruch 19.

Gemäß dem erfindungsgemäßen Verfahren ist vorgesehen, dass zunächst eine Faserflorbahn aus einem mehrschichtigen Nonwovenmaterial mit zumindest einer kardierten Stapelfaserlage und eine auf der Stapelfaserlage angeordneten Speicherlage gebildet wird, wobei die Speicherlage zumindest Zellstofffasern und die zumindest eine Stapelfaserlage zumindest teilweise thermoplastische und damit schmelzbare Stapelfasern aufweist. Im Anschluss wird sodann die Faserflorbahn bevorzugt beidseitig mit Flüssigkeitsstrahlen beaufschlagt, wodurch die Fasern des mehrschichtigen Nonwovenmaterials miteinander vermischt und verschlungen werden und wobei zugleich der Faserflorbahn eine Oberflächenstruktur eingeprägt wird.

Durch die Prägung wird der Nonwovenbahn eine dreidimensionale Struktur verliehen, welche sich durch stärker komprimierte und weniger bzw. nichtkomprimierte Bereiche auszeichnet. Entsprechend weist die Nonwovenbahn Bereiche mit einer höheren und Bereiche mit einer niedrigeren Flächendichte auf. Da sich in den stärker komprimierten Bereichen auch die Stapelfasern stärker miteinander mechanisch verhaken, zeichnen sich diese Bereiche auch durch eine erhöhte Festigkeit aus. Dieser Effekt wird durch das anschließende zumindest teilweise Verschmelzen der Stapelfasern noch erhöht. Die Prägung ist daher wesentlich für die Stabilisierung der Nonwovenbahn.

Darüber hinaus wird durch das Prägen auch die Oberfläche des Nonwovenmaterials vergrößert und die Rauheit erhöht. Grundsätzlich sind unterschiedliche Prägemuster geeignet. Neben punktförmigen und gitterförmigen Einprägungen kann das Nonwovenmaterial insbesondere auch mit linienförmigen oder wellenförmigen Einprägungen versehen sein.

Sodann erfolgt die Beaufschlagung der Faserflorbahn mit Hitze, wodurch die thermoplastischen Stapelfasern zumindest teilweise aufschmelzen und nach einer anschließenden Abkühlung die Faserflorbahn zu einer Nonwovenbahn verfestigen. Es versteht sich im Rahmen der Erfindung, dass bereits die Nonwovenbahn ein erfindungsgemäßes Nonwovenelement bildet, wobei abschließend einzelne zugeschnittene Nonwovenelemente aus der Nonwovenbahn abgetrennt werden können. Diese Beaufschlagung mit Hitze kann bevorzugt im Zuge eines sogenannten Air-Through-Bondings erfolgen. Hierbei durchfährt die Nonwovenbahn einen Heizofen und wird dort mit heißer Luft beaufschlagt. Ein derartiger Heizofen ist üblicherweise bei der Herstellung von Nonwoven vorgesehen, um eine Verfestigung der einzelnen Fasern zu bewirken.

Somit ist vorgesehen, dass das durch das erfindungsgemäße Verfahren hergestellte Nonwovenelement zumindest eine kardierte Stapelfaserlage aufweist, wodurch sich einerseits eine besonders weiche Oberfläche ergibt und zugleich ein sehr flexibler Einsatz von verschiedenen Fasermaterialien möglich ist. Zugleich bedarf es bei der Verwendung von Stapelfasern aber im besonderen Maße einer Verfestigung, da im Vergleich zu Endlosfilamenten die Zugfestigkeit wesentlich geringer ist. Dies wird erreicht durch die zweistufige Verfestigung einerseits mit Flüssigkeitsstrahlen, durch die sich die Stapelfasern der Stapelfaserlage und der Speicherlage miteinander verschlingen und andererseits durch die Beaufschlagung von Hitze, wodurch aneinandergrenzende Stapelfasern eine stoffschlüssige Verbindung eingehen.

Bevorzugt weist die Speicherlage nicht nur Zellstofffasern, sondern zugleich auch Stapelfasern, insbesondere Stapelfasern aus einem thermoplastischen Material, auf. Durch die Einbeziehung von Stapelfasern aus einem thermoplastischen Material kann die Speicherlage im besonderen Maße mit der zumindest einen Stapelfaserlage verbunden werden. Hierdurch erfolgt eine besonders gute Stabilisierung der Speicherschicht und Anbindung an die Stapelfaserlage.

Bevorzugt sind die Stapelfasern aus dem thermoplastischen Material ausgewählt und abgestimmt auf die in den Stapelfaserlagen verwendeten Stapelfasern, sodass im Zuge der Aufschmelzung das aufgeschmolzene thermoplastische Material jeweils in die angrenzenden Lagen eindringen kann und somit eine ausreichende Anbindung sichergestellt wird. Die Zellstofffasern in der Speicherschicht weisen bevorzugt eine Länge zwischen 4 und 10 mm, besonders bevorzugt zwischen 6 und 8 mm, auf.

Gemäß einer bevorzugten Weiterbildung ist vorgesehen, dass das mehrschichtige Nonwovenmaterial eine weitere Faserlage aufweist, wobei die Speicherlage zwischen der Stapelfaserlage und der weiteren Faserlage angeordnet ist, wobei auch die weitere Faserlage zumindest teilweise aus thermoplastischen Fasern gebildet sind. Hierdurch wird einerseits die Speicherlage zwischen zwei äußeren Faserlagen eingekapselt, wobei zugleich durch die Verwendung von thermoplastischen Fasern in beiden äußeren Faserlagen sowie in der Speicherlage ein ausreichend fester Verbund geschaffen werden kann. Die beiden äußeren Faserlagen können hierbei grundsätzlich identisch ausgebildet sein. Bevorzugt ist jedoch eine Ausgestaltung, bei der sich die beiden äußeren Faserlagen hinsichtlich ihres Flächengewichts und/oder der verwendeten Fasern und/oder hinsichtlich der verwendeten Fasermaterialien voneinander unterscheiden. Hierdurch können die einzelnen Oberflächen der Nonwovenelemente für unterschiedliche Anwendungszwecke ausgebildet werden.

Eine Möglichkeit besteht beispielsweise darin, dass eine weitere Faserlage als Spinnfaserlage aus Endlosfilamenten gebildet ist, sodass die eine Seite des Nonwovenelements im ausreichenden Maße für die Stabilität und Zugfestigkeit und die andere Seite für eine besonders weiche Beschaffenheit ausgebildet ist. Hierdurch werden die Eigenschaften der unterschiedlichen Vliesstoffarten in vorteilhafter Weise miteinander kombiniert. Alternativ können aber auch beide äußeren Faserlagen als kardierte Stapelfaserlage gebildet sein, wodurch sich die Flexibilität hinsichtlich des Einsatzes unterschiedlicher Fasermaterialien wesentlich erhöht.

Unabhängig von der konkreten Ausgestaltung beider Faserflorlagen erfolgt die Herstellung der Faserflorbahn in einem mehrstufigen Prozess, wobei zunächst eine erste Faserlage entweder in einem Spinnvliesverfahren oder durch Kardieren von Stapelfasern gebildet wird. Auf diese erste Faserlage wird sodann die Speicherlage in einem sogenannten Airlaid-Verfahren angeordnet, wobei einzelne Stapelfasern und Zellstofffasern lose auf der ersten Faserlage aufgelegt werden. In diesem Zusammenhang meint der Begriff Zellstofffasern Fasern aus einem Zellstoffmaterial, z. B. Papier, welche wesentlich kürzer ausgebildet sind als die Stapelfasern. Im Gegensatz zu einem Prozess in einer Kardiermaschine werden die Fasern der Textilschicht somit nicht in Produktionsrichtung ausgerichtet, sondern nur lose aufgelegt. Sodann kann eine weitere Faserlage auf der Textilschicht angeordnet werden, wobei es sich im Falle einer ersten Faserlage aus Spinnvlies zwingend um eine Faserlage aus kardierten Stapelfasern handelt, welche ausgehend von einer Kardiermaschine zugeführt wird. Sofern die erste Faserlage bereits eine Stapelfaserlage ist, kann auf diesen Herstellungsschritt im Rahmen der Erfindung grundsätzlich verzichtet werden. Allerdings ist es auch dann bevorzugt, wenn die Speicherlage zwischen zwei kardierten Stapelfaserlagen angeordnet ist.

Gemäß einer bevorzugten Weiterbildung der Erfindung sind die thermoplastischen Stapelfasern in der zumindest einen Stapelfaserlage aus einem thermoplastischen Material und/oder als Zweikomponentenfasern mit einem Kern und einem Mantel aus einem thermoplastischen Material gebildet. Der Kern ist hingegen aus einem nicht-thermoplastischen Material oder aus einem thermoplastischen Material gebildet, welches eine höhere Schmelztemperatur aufweist als die des thermoplastischen Materials des Mantels. Die Verwendung von Zweikomponentenfasern ist in diesem Zusammenhang von besonderem Vorteil, da einerseits in einem ausreichenden Maße festgelegt werden kann, wie weit ein Aufschmelzen bzw. wie weit eine stoffschlüssige Verbindung zwischen einzelnen Stapelfasern zweckmäßig ist, während zugleich der Kern seine Struktur beibehält und somit auch nach dem Aufschmelzen eine ausreichende Stabilität sichergestellt wird. Grundsätzlich können die thermoplastischen Stapelfasern vollständig als Zweikomponentenfasern ausgebildet sein.

Die Stapelfasern können darüber hinaus auch mit nicht-runden Querschnitten ausgebildet sein (shaped fibers). Aufgrund des nicht-runden Querschnittes wird die Oberfläche der Fasern, bei gleichem Flächengewicht des Vliessstoffes und gleichem Längengewicht der Faser, vergrößert. Auch die optische Erscheinung kann von besonderem Vorteil sein.

Um sicherzustellen, dass die Stapelfaserlagen im ausreichenden Maße verfestigt werden, ist vorgesehen, dass der Anteil der thermoplastischen Stapelfasern in jeweils einer der Stapelfaserlagen zumindest 10 Gew.-%, bevorzugt zumindest 15 Gew.-%, besonders bevorzugt zumindest 20 Gew.-% beträgt. Bei der Einbindung einer Spinnfaserlage sind die dort verwendeten Endlosfilamente ohnehin ausschließlich aus einem thermoplastischen Material gebildet.

Bei der Verwendung von Zweikomponentenfasern in der zumindest einen Stapelfaserlage ist bevorzugt vorgesehen, dass der Kern aus einem Material gebildet ist, welches bei einer Temperatur von 170 °C eine Längenänderung, insbesondere eine Schrumpfung, von weniger als 10 % aufweist. Dies stellt sicher, dass der Kern auch bei Einwirkung von Hitze ausreichend stabil bleibt und sich damit die Struktur des Nonwovenmaterials nicht im übermäßigen Maße verändert. Alternativ kann es allerdings auch explizit vorgesehen sein, dass die Fasern einer gewissen Schrumpfung unterliegen, um hierdurch die abrasive Wirkung zu erhöhen.

Zugleich weist das thermoplastische Material der Stapelfasern in der zumindest einen Stapelfaserlage und/oder in der Speicherlage eine Schmelztemperatur von weniger als 170 °C auf. Alternativ kann das thermoplastische Material in der Speicherlage auch eine geringere Schmelztemperatur als in den Stapelfaserlagen aufweisen. Insbesondere kann die Temperaturdifferenz zwischen 10 und 50 K, bevorzugt zwischen 20 und 40 K betragen.

Für das thermoplastische Material in den Stapelfaserlagen und/oder in der Speicherlage kommen insbesondere Polyolefine, insbesondere ausgewählt aus der Gruppe Polyethylen (PE) oder Polypropylen (PP), oder Thermoplaste aus nachwachsenden Rohstoffen, insbesondere ausgewählt aus der Gruppe Polylactid-Acid (PLA) und Polyhydroxy-Alkanoate (PHA), in Betracht. Beispielsweise können alle thermoplastischen Materialien dasselbe thermoplastische Material aufweisen. Um insgesamt geringe Herstellungskosten zu ermöglichen, sind auch die Endlosfilamente der Spinnfaserlage vorzugsweise aus den zuvor genannten Materialien gebildet, wobei insbesondere ein Polyolefin, vorzugsweise Polypropylen, besonders geeignet ist. Bei der Verwendung von Zweikomponentenfasern kann auch der Kern aus Polylactid-Acid (PLA) oder Polyhydroxy-Alkanoate (PHA) gebildet sein, da diese Materialien mit unterschiedlichen thermischen Eigenschaften erhältlich sind. Beispielsweise können der Kern aus PLA1 und der Mantel aus PLA2 gebildet sein, wobei PLA1 einen höheren Schmelzpunkt aufweist als PLA2.

Dadurch, dass zumindest eine Stapelfaserlage aus kardierten Stapelfasern vorgesehen ist, können in dieser Faserlage auch Stapelfasern vorgesehen sein, welche nicht aus einem thermoplastischen Material gebildet sind. Hierbei handelt es sich beispielsweise um Viskose oder aber auch um Naturfasern. Insbesondere ist das Material der Naturfasern ausgewählt aus der Gruppe Baumwolle, Wolle, Leinen, Hanf, Zellulose und Flachs. Durch die Verwendung von Naturfasern sind die hieraus gebildeten Nonwovenelemente besonders nachhaltig herstellbar. Darüber hinaus lassen sich die Nonwovenelemente auch gut biologisch recycelt. Eine weitere Erhöhung der Nachhaltigkeit kann dadurch erreicht werden, dass die Fasern zumindest teilweise aus recycelten Materialien gebildet sind.

Das erfindungsgemäße Verfahren kann auch vorsehen, dass die Nonwovenbahn auf einer Seite thermisch geglättet wird. Hierbei handelt es sich um ein Verfahren, bei der die Nonwovenbahn auf nur einer Seite entlang einer beheizten Oberfläche geführt wird und wobei die Nonwovenbahn zu einem gewissen Maße auf die beheizte Oberfläche gepresst wird. Dieses Verfahren kann verglichen werden mit einem Bügelverfahren, wodurch die Nonwovenbahn zusätzlich auf einer Seite verfestigt wird und sich hierdurch unterschiedliche Verfestigungsstufen auf beiden Seiten der Nonwovenbahn einstellen lassen. Darüber hinaus werden die thermoplastischen Stapelfasern zusätzlich zu einem gewissen Maße aufgeschmolzen und hierdurch die eine Seite der Nonwovenbahn geglättet.

Das erfindungsgemäße Verfahren kann darüber hinaus dadurch ergänzt werden, dass das Nonwovenmaterial mit zusätzlichen chemischen Flüssigkeiten besprüht oder das Nonwovenmaterial in diesen chemischen Flüssigkeiten getränkt wird. Beispielsweise kann es sich hierbei eine Salzlösung handeln, wodurch die Absorption von kationischen Desinfektionsmitteln reduziert wird. Darüber hinaus ist auch der Einsatz von Bindemitteln denkbar, die die Fusselbildung wesentlich verringern. Schließlich können auch sogenannte grenzflächenaktive Substanzen zum Einsatz kommen. Diese ermöglichen eine Ausgestaltung des Nonwovenelements, das zunächst in einem trockenen Zustand vorliegt und eine Flüssigkeit in der Speicherlage erst beim Einsatz nach außen tritt. Hygieneartikel welche aus einem solchen Material gebildet werden, werden gewöhnlich auch als "dry-untiluse-wipes" bezeichnet.

Gegenstand der Erfindung ist ferner ein Nonwovenelement für Hygieneartikel, welches insbesondere erhältlich ist durch das erfindungsgemäße Verfahren. Dieses Nonwovenelement weist ein mit einer Oberflächenstruktur eingeprägtes, mehrschichtiges Nonwovenmaterial mit zumindest einer kardierten Stapelfaserschicht und einer auf der Stapelfaserschicht angeordneten Speicherschicht aus Stapelfasern und Zellstofffasern auf, wobei die Fasern des Nonwovenmaterials miteinander verschlungen und zumindest teilweise stoffschlüssig miteinander verbunden sind. Die stoffschlüssige Verbindung kann durch ein teilweises Verschmelzen der Fasern miteinander erreicht werden.

Grundsätzlich gelten für dieses Nonwovenelement alle gegenständlichen Merkmale, welche auch im Zusammenhang mit dem erfindungsgemäßen Verfahren genannt sind und insbesondere die Ausgestaltung des Nonwovenmaterials betreffen. Dies beinhaltet insbesondere die Anordnung der einzelnen Faserschichten bzw. Faserlagen sowie auch die verwendeten Materialien.

Insbesondere ist vorgesehen, dass auch bei dem Nonwovenelement die Speicherschicht zwischen zwei äußeren Faserschichten angeordnet ist, wobei es sich entweder in beiden Fällen um kardierte Stapelfaserschichten handelt oder wobei eine der Faserschichten als Spinnfaserschicht aus Endlosfilamenten gebildet ist.

Bevorzugt ist vorgesehen, dass zumindest eine der Faserschichten Fasern aus einem lipophilen und/oder hydrophilen Material aufweist. Auch Fasern mit einer Beschichtung aus einem lipophilen und/oder hydrophilen Material kommen in Betracht. Unter hydrophilen Materialien werden diesbezüglich Materialien verstanden, welche in Wasser löslich sind. Hingegen sind lipophile Materialien fettlöslich. Durch diese Eigenschaften lassen sich besonders gut fetthaltige, aber auch nicht-fetthaltige Schmutzpartikel aufnehmen.

Das Nonwovenelement zeichnet sich darüber hinaus durch ein Flächengewicht zwischen 40 und 150 g/m² aus. Die Speicherschicht zeichnet sich hierbei insbesondere durch ein Flächengewicht zwischen 10 und 70 g/m² bevorzugt zwischen 20 und 50 g/m², aus, während die beiden äußeren Faserschichten jeweils ein Flächengewicht zwischen 5 und 70 g/m², bevorzugt zwischen 10 und 50 g/m² aufweisen.

Gegenstand der Erfindung ist ferner ein Hygieneartikel, insbesondere ein Hygienetuch, mit oder aus einem erfindungsgemäßen Nonwovenelement.

Bevorzugt kann dieses Nonwovenelement zumindest teilweise mit einer Reinigungsflüssigkeit getränkt sein.

Darüber hinaus kann das Nonwovenelement auf einer Seite mit einer Struktur, insbesondere einer geprägten Struktur, versehen sein. Hierbei weist das Nonwovenelement dann Bereiche mit einer größeren Härte bzw. Kompression auf, wodurch eine zusätzliche Stabilisierung erfolgt. Zugleich weist die Oberfläche eine höhere Rauheit auf, wodurch die Reinigungsleistung verbessert werden kann. Die strukturierte Seite bildet demnach bevorzugt die Reinigungsseite.

Weiter ist bevorzugt vorgesehen, dass Anschlusselemente zur mechanischen Befestigung an einer Reinigungseinheit vorgesehen sind. Bei dieser Reinigungseinheit kann es sich beispielsweise um einen Staubsaugroboter, einen Staubsauger, einen Stiel, einen Handgriff oder dergleichen handeln.

Im Folgenden wird die Erfindung anhand des Ausgangsbeispiels näher erläutert. Es zeigen:
- Fig. 1, 2: erfindungsgemäße Verfahren zur Herstellung eines Nonwovenelements,
- Fig. 3: ein Nonwovenelement hergestellt mit dem Verfahren gemäß Fig. 1,
- Fig. 4: ein Nonwovenelement hergestellt mit dem Verfahren gemäß Fig. 2,
- Fig. 5: das Nonwovenelement gemäß Fig. 3.

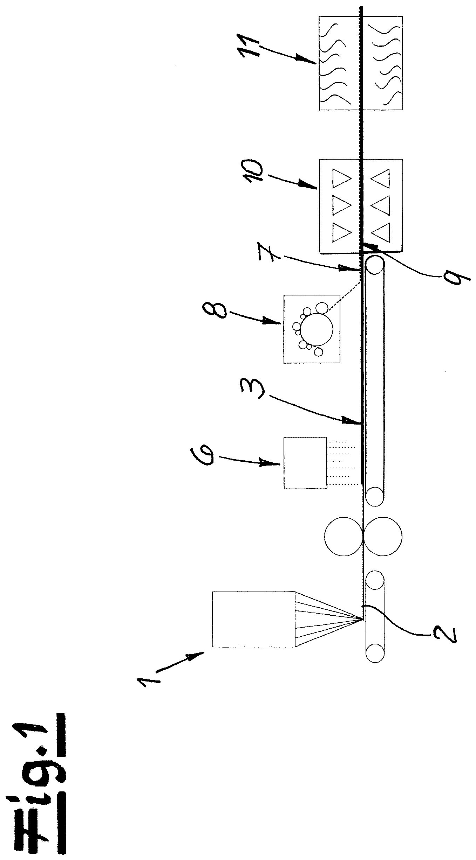

Die Fig. 1 zeigt ein erfindungsgemäßes Verfahren, wobei zunächst mit Hilfe eines Spinnfaserextruders 1 eine erste Faserlage 2 als Spinnfaserlage gebildet wird, wobei die erste Faserlage 2 somit eine Vielzahl von Endlosfilamenten 20 aufweist. Die Endlosfilamente sind aus Polypropylen gebildet, wobei die Spinnfaserlage insgesamt mit einem Flächengewicht zwischen 10 und 18 g/m² gebildet wird.

In einem weiteren Prozessschritt wird sodann eine Speicherschicht 3 aus Stapelfasern 4 und Zellstofffasern 5 auf die erste Faserlage 2 aufgelegt. Dies erfolgt in einem sogenannten Airlaid-Verfahren mit einer Airlaidmaschine 6, welche die Stapelfasern 4 und die Zellstofffasern 5 der Speicherschicht 3 lose auf der ersten Faserlage 2 auflegt.

Sodann erfolgt das Einbringen einer zweiten Faserlage 7, welche als kardierte Stapelfaserlage mit einer Vielzahl von Stapelfasern ausgebildet ist und derart angeordnet wird, dass die Speicherschicht 3 zwischen der ersten Faserlage 2 und der zweiten Faserlage 7 angeordnet ist. Die einzelnen Stapelfasern der zweiten Faserlage 7 werden in diesem Zusammenhang über eine Kardiermaschine 8 ausgerichtet und als zusammenhängende Faserlage 7 zugeführt.

Insgesamt bildet sich somit ein mehrschichtiges Nonwovenmaterial aus zwei Faserlagen 2, 7 sowie einer zwischen den Faserlagen 2, 7 angeordneten Speicherschicht 3 aus, wobei zumindest die zweite Faserlage 7 als Stapelfaserlage und auch die Speicherschicht thermoplastische Stapelfasern 4 aufweist, welche entsprechend zumindest teilweise aus einem thermoplastischen Material gebildet sind.

In einem anschließenden Verfahrensschritt wird die so ausgebildete Faserflorbahn 9 aus einem mehrschichtigen Nonwovenmaterial in einer Vorrichtung 10 mit Flüssigkeitsstrahlen derart beaufschlagt, dass sich die Fasern der Faserflorbahn 9 miteinander vermischen und verschlingen. Zugleich wird der Faserflorbahn 9 eine Oberflächenstruktur eingeprägt. Die zunächst relativ lose aneinander liegenden Lagen des mehrschichtigen Nonwovenmaterials werden durch mechanische Verschlingung der Fasern miteinander verbunden und dadurch die Faserflorbahn 9 verfestigt.

In einem nächsten Schritt kann das so verfestigte Nonwovenmaterial der Faserflorbahn 9 einem Ofen 11 zugeführt werden, wodurch die Faserflorbahn 9 mit heißer Luft bzw. mit Hitze beansprucht wird. Hierbei ist es wesentlich, dass die Endlosfilamente der ersten Faserlage 2 als auch die thermoplastischen Stapelfasern 4 der zweiten Faserlage 7 und der Speicherschicht 3 zumindest teilweise aus einem thermoplastischen Material gebildet sind. Unter Einwirkung von Hitze schmelzen die Fasern auf und verbinden sich stoffschlüssig miteinander. Hierdurch wird insbesondere die Speicherschicht 3 zwischen der ersten und der zweiten Faserlage 2, 7 stabilisiert und die mehrschichtige Struktur der Faserflorbahn 9 festgelegt. Der Ofen 11 wird hierzu mit einer Temperatur betrieben, welche zumindest 170 °C beträgt, wobei sich die Temperatur nach der Schmelztemperatur der verschiedenen Fasermaterialien richtet.

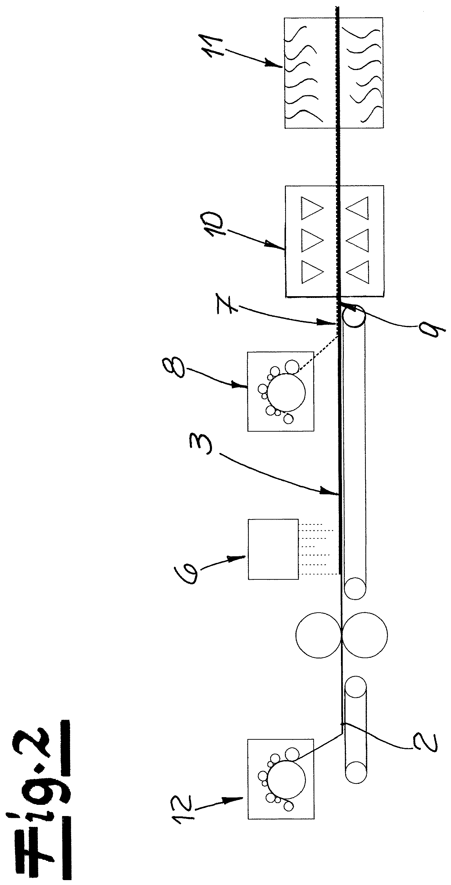

Die Fig. 2 zeigt ein alternatives Verfahren, wobei anstelle eines Spinnfaserextruders 1 eine weitere zweite Kardiermaschine 12 vorgesehen ist, die die erste Faserlage 2 ebenfalls als Stapelfaserlage ausbildet. Die weiteren Prozessschritte stimmen dann mit denen der Fig. 1 überein.

Somit unterschieden sich die Nonwovenbahnen 9 dadurch, dass gemäß der Fig. 2 ausschließlich Stapelfaserlagen vorgesehen sind, während gemäß der Fig. 1 eine Faserlage als Spinnfaserlage ausgebildet ist.

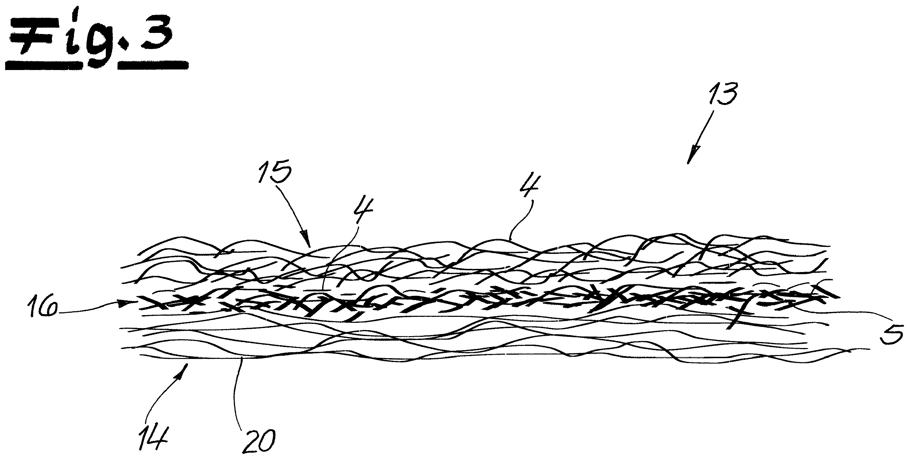

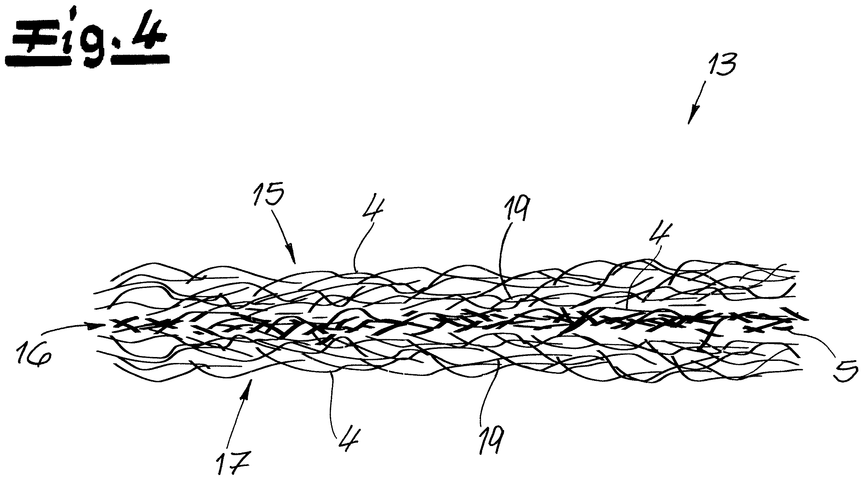

Die Fig. 3 zeigt eine Nonwovenelement 13, welches mit einem Verfahren gemäß der Fig. 1 durch Abtrennen aus der Nonwovenbahn 9 gebildet wurde. Die einzelnen Verfestigungsschritte sind sowohl in der Fig. 3 als auch in der Fig. 4 zunächst nicht dargestellt. Das Nonwovenelement 13 zeichnet sich somit durch einen dreischichtigen Aufbau mit Spinnfaserschicht 14 und einer Stapelfaserschicht 15 aus, welche aus kardierten Stapelfasern gebildet ist. Zwischen der Stapelfaserschicht 15 und der Spinnfaserschicht 14 ist eine Speicherschicht 16 angeordnet, welche aus thermoplastischen Stapelfasern 4 und Zellstofffasern 5 gebildet ist. Gemäß der Fig. 4 sind zwei Stapelfaserschichten 15, 17 vorgesehen, welche aus den Stapelfaserlagen gemäß der Fig. 2 hervorgehen. In beiden Fällen sind in den Stapelfaserschichten 15, 17 thermoplastische Stapelfasern 4 vorgesehen, welche als Zweikomponentenfasern mit einem Kern aus einem nicht-thermoplastischen Material und einem Mantel aus einem thermoplastischen Material gebildet sind. Grundsätzlich reicht es aber auch aus, wenn lediglich eine der Spinnfaserlagen 15, 17 thermoplastische Stapelfasern 4 aufweist. Auch die Endlosfilamente 20 der Spinnfaserschicht 14 sind aus einem thermoplastischen Material gebildet.

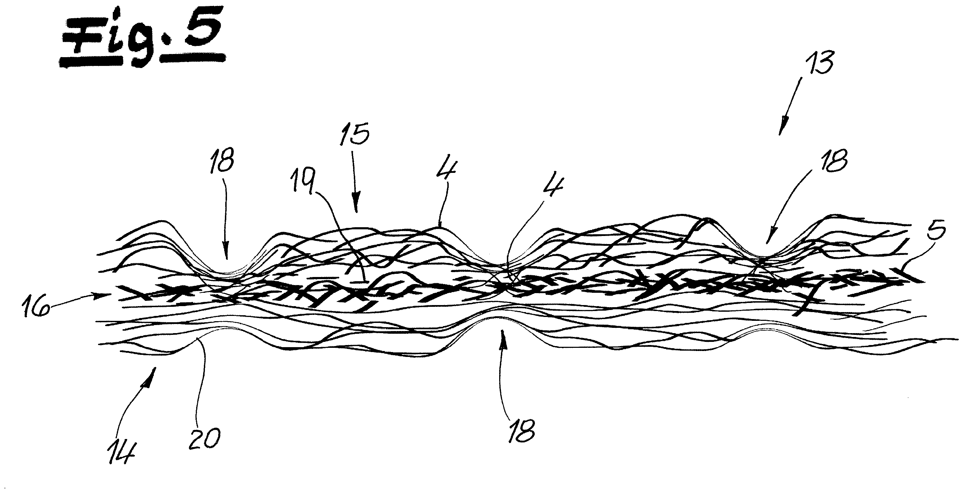

Die Fig. 5 zeigt ein erfindungsgemäßes Nonwovenelement 13 gemäß der Fig. 3, wobei nunmehr zusätzlich eine Oberflächenstruktur in Form von Einbuchtungen 18 sichtbar ist. Darüber hinaus wurden die einzelnen Fasern auch mittels Flüssigkeitsstrahlen miteinander verbunden.

Darüber hinaus ist erfindungsgemäß vorgesehen, dass die Speicherschicht 16 zumindest zum Teil aus nicht-thermoplastischen Stapelfasern 19, insbesondere aus Naturfasern, gebildet ist. Bei diesen Naturfasern kann es sich beispielsweise um Baumwolle, Wolle, Leinen, Hanf, Flachs oder dergleichen handeln.

### Beispiel 1:

Im Rahmen eines ersten Ausführungsbeispiels wurde ein Nonwovenelement 13 gebildet mit einer äußeren Spinnfaserschicht 14 aus Polypropylen und einem Flächengewicht von 12 g/m². Die Speicherschicht 16 weist ein Flächengewicht von 28 g/m² auf und ist eingeschlossen von einer Stapelfaserschicht 15 aus kardierten thermoplastischen Stapelfasern 4 mit einem Kern aus Polyethylenterephthalat und einem Mantel aus Polyethylen. Die Stapelfasern weisen eine Feinheit von 1,7 dtex auf. Das Flächengewicht der Stapelfaserschicht 15 beträgt 20 g/m². Das Nonwovenelement 13 weist darüber hinaus eine eingeprägte Oberflächenstruktur in Form von punktförmigen Vertiefungen auf.

### Beispiel 2:

Gemäß eines weiteren Ausführungsbeispiels wurde ein Nonwovenelement 13 gebildet mit einer äußeren Spinnfaserschicht 14 aus Polypropylen und einem Flächengewicht von 15 g/m². Die Speicherschicht 16 besteht aus Lyocell-Stapelfasern und weist ein Flächengewicht von 50 g/m² auf. Bei Lyocell-Stapelfasern handelt es sich um Regeneratfasern aus Zellulose. Die Speicherschicht 16 ist eingeschlossen von einer Stapelfaserschicht 15 aus kardierten thermoplastischen Stapelfasern 4 mit einem Kern aus Polyethylenterephthalat und einem Mantel aus Polyethylen. Die Stapelfasern weisen eine Feinheit von 3,4 dtex auf. Das Flächengewicht der Stapelfaserschicht 15 beträgt 50 g/m². Das Nonwovenelement 13 weist darüber hinaus eine eingeprägte Oberflächenstruktur in Form von punktförmigen Vertiefungen auf.

### Beispiel 3:

Gemäß einem dritten Ausführungsbeispiel sind zwei Stapelfaserschichten 15, 17 vorgesehen. Die eine Stapelfaserschicht 15 ist aus Stapelfasern 4 aus Polyethylenterephthalat gebildet. Die Stapelfasern weisen eine Feinheit von 1,7 dtex auf. Das Flächengewicht der Stapelfaserschicht 15 beträgt 15 g/m². Die Speicherschicht 16 entspricht der des Beispiels 1 mit einem Flächengewicht von 30 g/m². Die zweite Stapelfaserschicht 17 ist aus thermoplastischen Stapelfasern 4 mit einem Kern aus Polyethylenterephthalat und einem Mantel aus Polyethylen gebildet. Die Feinheit beträgt 2,2 dtex und das Flächengewicht 15 g/m².

### Beispiel 4:

Gemäß einem vierten Ausführungsbeispiel sind zwei Stapelfaserschichten 15, 17 vorgesehen. Die eine Stapelfaserschicht 15 ist aus thermoplastischen Stapelfasern 4 mit einem Kern aus einem ersten Polylactid und einem Mantel aus einem zweiten Polylactid gebildet, wobei die Schmelztemperatur des ersten Polylactids 30 K höher liegt als die des zweiten Polylactids. Die Stapelfasern weisen eine Feinheit von 3,4 dtex auf. Das Flächengewicht der Stapelfaserschicht 15 beträgt 25 g/m². Die weitere Stapelfaserschicht 17 ist fast identisch ausgebildet, wobei lediglich die Feinheit 1,7 dtex beträgt. Die Speicherschicht 16 entspricht der des Beispiels 1, allerdings mit einem Flächengewicht von 50 g/m².

### Beispiel 5:

Gemäß einem fünften Ausführungsbeispiel sind zwei Stapelfaserschichten 15, 17 vorgesehen. Die erste Stapelfaserschicht 15 und die zweite Stapelfaserschicht 17 sind zu einem Anteil von 50 % aus thermoplastischen Stapelfasern 4 mit einem Kern aus Polyethylenterephthalat und einem Mantel aus Polyethylen und zu einem weiteren Anteil von 50 % aus thermoplastischen Stapelfasern 4 aus Polyethylenterephthalat gebildet. Die Feinheit beträgt 1,7 dtex und das Flächengewicht 20 g/m². Die Speicherschicht 16 entspricht der des Beispiels 1, allerdings mit einem Flächengewicht von 30 g/m².

### Beispiel 6:

Gemäß einem sechsten Ausführungsbeispiel sind zwei Stapelfaserschichten 15, 17 vorgesehen. Die erste Stapelfaserschicht 15 ist aus Stapelfasern aus Viskose mit einer Feinheit von 1,7 dtex und die zweite Stapelfaserschicht 17 aus Stapelfasern mit einem Kern aus Polyethylenterephthalat und einem Mantel aus Polyethylen mit einer Feinheit von 3,4 dtex gebildet. Bei Viskose handelt es sich um regenerierte Zellulose. Das Flächengewicht beträgt 15 g/m² für die erste Stapelfaserschicht 15 und 20 g/m² für die zweite Stapelfaserschicht 17. Die Speicherschicht 16 entspricht der des Beispiels 1, allerdings mit einem Flächengewicht von 30 g/m².

### Beispiel 7:

Gemäß einem siebten Ausführungsbeispiel sind zwei Stapelfaserschichten 15, 17 aus Stapelfasern aus Viskose mit einer Feinheit von 1,7 dtex gebildet. Das Flächengewicht beträgt jeweils 20 g/m². Die Speicherschicht 16 ist aus einem Gemisch aus Tyvek-Fasern (HDPE) und Zelluloseflocken mit einem Flächengewicht von 40 g/m² gebildet.

In allen zuvor genannten Beispielen bildet jeweils die zweite Stapelfaserschicht 17 oder die Spinnfaserschicht 14 eine Seite eines Nonwovenelements 13, welche eine Reinigungsseite eines Hygieneartikels, z. B. eines Reinigungstuches, bildet. Hierzu können die in diesen Schichten verwendeten Fasern eine größere Dicke aufweisen als die Stapelfasern der Stapelfaserschicht 15. Durch die dickeren Fasern wird die abrasive Wirkung im Zuge der Reinigung erhöht. Nachfolgend sind verschiedene Hygieneprodukte aufgeführt, welche beispielhaft aus den zuvor genannten Nonwovenelementen 13 gebildet wurden.

Aus den zuvor genannten Nonwovenelementen 13 können unterschiedliche Hygieneprodukte hergestellt werden, wobei nachfolgend exemplarisch verschiedene Hygienetücher aufgeführt sind.

### Beispiel 8:

Hygienetücher zur Nassreinigung, wobei der Anteil an hydrophilen und lipophilen Fasern gleichgroß ist, um einerseits genügend Reinigungsflüssigkeit aufnehmen zu können und um andererseits abgetragenes Fett und Schmutz aufnehmen zu können. Darüber hinaus weist das Hygienetuch Bereiche mit hoher Härte auf. Dies kann beispielsweise durch eine Heißprägung erreicht werden. Darüber hinaus kann das Hygienetuch auch Anschlusselemente zur mechanischen Befestigung an einer Reinigungseinheit aufweisen.

### Beispiel 9:

Das Hygienetuch aus dem Beispiel 8, welches allerdings zur Trockenreinigung ausgebildet ist und entsprechend nicht mit einer Reinigungsflüssigkeit getränkt ist. Hierdurch lassen sich insbesondere Flüssigkeiten im Zuge der Reinigung besonders gut aufnehmen.

### Beispiel 10:

Ein Hygienetuch mit unterschiedlichen Seiten, wobei eine erste Seite vornehmlich mit hydrophilen Fasern gebildet ist und wobei die zweite Seite die eigentliche Reinigungsseite darstellt. Über die erste Seite liegt das Nonwovenelement 13 an einem Reinigungsflüssigkeitsreservoir an, welches bei Druckausübung über die erste Seite einen Transport von Reinigungsflüssigkeit zur zweiten Seite ermöglicht. Die Reinigungsseite ist auch hier mit Bereichen hoher Härte ausgebildet. Diese Bereiche erhöhen die Effizienz und die Wirksamkeit der Reinigung, während die erste Seite den kapillaren Transport von Reinigungsflüssigkeit ermöglicht.

Auch hier kann das Hygienetuch Anschlusselemente zur mechanischen Befestigung an einer Reinigungseinheit aufweisen.

## Patentansprüche

1. Verfahren zur Herstellung eine Nonwovenelements (13) für Hygieneartikel, umfassend zumindest die Schritte:
- Bildung einer Faserflorbahn (9) aus einem mehrschichtigen Nonwovenmaterial mit zumindest einer kardierten Stapelfaserlage und einer auf der Stapelfaserlage angeordneten Speicherlage (7), welche Zellstofffasern (5) aufweist, wobei zumindest ein Teil der Stapelfasern (4) der Stapelfaserlage aus einem thermoplastisch Material gebildet sind,
- Beaufschlagung der Faserflorbahn (9) mit Flüssigkeitsstrahlen, wodurch die Fasern des mehrschichtigen Nonwovenmaterials miteinander vermischt und verschlungen werden und der Faserflorbahn (9) eine Oberflächenstruktur eingeprägt wird,
- Beaufschlagung der Faserflorbahn (9) mit Hitze, wodurch die thermoplastischen Stapelfasern (4) zumindest teilweise aufschmelzen und die Faserflorbahn (9) zu einer Nonwovenbahn verfestigen.

2. Verfahren nach Anspruch 1, wobei die Speicherlage (7) zusätzlich Stapelfasern, insbesondere aus einem thermoplastischen Material, aufweist.

3. Verfahren nach Anspruch 1 oder 2, wobei das mehrschichtige Nonwovenmaterial eine weitere Faserlage aufweist, wobei die Speicherlage (7) zwischen der Stapelfaserlage und der weiteren Faserlage angeordnet ist, wobei die weitere Faserlage zumindest teilweise aus thermoplastischen Stapelfasern (4) gebildet ist.

4. Verfahren nach einem der Ansprüche 1 bis 3, wobei auch die weitere Faserlage als kardierte Stapelfaserlage oder als Spinnvlieslage aus Endlosfilamenten gebildet ist.

5. Verfahren nach einem der Ansprüche 1 bis 4, wobei die thermoplastischen Stapelfasern (4) in der zumindest einen Stapelfaserlage zumindest teilweise aus einem thermoplastischen Material oder als Zweikomponentenfasern mit einem Kern und einem Mantel aus einem thermoplastischen Material gebildet sind.

6. Verfahren nach einem der Ansprüche 1 bis 5, wobei der Anteil der thermoplastischen Stapelfasern (4) in der zumindest einen Stapelfaserlage zumindest 20 Gew.-% beträgt.

7. Verfahren nach Anspruch 5 oder 6, wobei der Kern aus einem Material gebildet ist, welches bei einer Temperatur von 170 °C eine Längenänderung von weniger als 10 % aufweist.

8. Verfahren nach Anspruch 1 bis 7, wobei das thermoplastische Material der thermoplastischen Stapelfasern (4) in der zumindest einen Stapelfaserlage und/oder in der Speicherlage (7) eine Schmelztemperatur von weniger als 170 °C aufweist.

9. Verfahren nach Anspruch 5 bis 8, wobei das thermoplastische Material der thermoplastischen Stapelfasern (4) in der zumindest einen Stapelfaserlage und/oder in der Speicherlage (7) ein Polyolefin, insbesondere ausgewählt aus der Gruppe Polyethylen oder Polypropylen, ist.

10. Verfahren nach einem der Ansprüche 5 bis 9, wobei die thermoplastischen Stapelfasern (4) der zumindest einen Stapelfaserlage und in der Speicherlage (7) dasselbe thermoplastische Material aufweisen.

11. Verfahren nach einem der Ansprüche 1 bis 10, wobei ein Teil der Stapelfasern (19) in der zumindest einen Stapelfaserlage aus einem nicht-thermoplastischen Material gebildet sind.

12. Verfahren nach Anspruch 11, wobei die aus einem nicht-thermoplastischen Material gebildeten Stapelfasern (19) Naturfasern, insbesondere ausgewählt aus der Gruppe Baumwolle, Wolle, Leinen, Hanf, Flachs, Zellulose, sind.

13. Verfahren nach Anspruch 1 bis 12, wobei die Nonwovenbahn (9) auf einer Seite thermisch geglättet wird.

14. Verfahren nach einem der Ansprüche 1 bis 13, wobei die Nonwovenbahn (9) ein Flächengewicht zwischen 45 und 150 g/m² aufweist.

15. Verfahren nach einem der Ansprüche 1 bis 14, wobei die Zellstofffasern (5) eine Länge zwischen 4 und 10 mm, bevorzugt zwischen 6 und 8 mm, aufweisen.

16. Nonwovenelement (13) für Hygieneartikel, insbesondere erhältlich durch ein Verfahren gemäß Anspruch 1 bis 15, aus einem mit einer Oberflächenstruktur eingeprägten, mehrschichtigen Nonwovenmaterial mit zumindest einer kardierten Stapelfaserschicht (15, 17) und einer auf der Stapelfaserschicht (15, 17) angeordneten Speicherschicht (16) aus Stapelfasern und Zellstofffasern (5), wobei die Fasern des Nonwovenmaterials miteinander verschlungen und die Stapelfasern zumindest teilweise stoffschlüssig miteinander verbunden sind.

17. Nonwovenelement (13) nach Anspruch 16, wobei die Speicherschicht (16) zwischen zwei Faserschichten (14, 15, 17) angeordnet ist.

18. Nonwovenelement (13) nach Anspruch 17, wobei die zwei Faserschichten (14, 15, 17) als kardierte Stapelfaserschichten (15, 17) ausgebildet sind oder wobei eine der Faserschichten (14, 15, 17) als Spinnvliesschicht (14) aus Endlosfilamenten (20) gebildet ist.

19. Nonwovenelement (13) nach einem der Ansprüche 16 bis 18, **gekennzeichnet durch** ein Flächengewicht zwischen 40 und 150 g/m².

20. Hygieneartikel, insbesondere Hygienetuch oder Feuchttuch, mit oder aus einem Nonwovenelement (13) gemäß Ansprüchen 16 bis 19.

21. Hygieneartikel nach Anspruch 20, wobei das Nonwovelement (13) zumindest teilweise mit einer Reinigungsflüssigkeit getränkt ist.

22. Hygieneartikel nach Anspruch 20 oder 21, wobei Anschlusselemente zur mechanischen Befestigung an einer Reinigungseinheit vorgesehen sind.
